# EUROPÄISCHE PATENTSCHRIFT

(11) **EP 0 746 308 B1**
(45) Veröffentlichungstag und Bekanntmachung des Hinweises auf die Patenterteilung: **15.05.2002**
(21) Anmeldenummer: 95908939.2
(22) Anmeldetag: 14.02.1995
(51) Int. Cl.: A61K 7/50

(54) **EMULSIONEN**
EMULSIONS
EMULSIONS

(30) Priorität: 22.02.1994 DE 4405510
(43) Veröffentlichungstag der Anmeldung: 11.12.1996
(73) Patentinhaber: Henkel Kommanditgesellschaft auf Aktien, 40191 Düsseldorf (DE)
(72) Erfinder: SEIDEL, Kurt, D-40589 Düsseldorf (DE); PRIEBE, Christian, D-42489 Wülfrath (DE); HOLLENBERG, Detlef, D-40699 Erkrath (DE)
(86) Internationale Anmeldenummer: EP9500533
(87) Internationale Veröffentlichungsnummer: WO9522313

(56) Entgegenhaltungen:
- EP-A- 0 512 270
- US-A- 3 625 706
- COSMETIC NEWS, Bd. 16, Dezember 1993 Seiten 408-414, PROSERPIO G. ET AL. 'from alkaline emusifiers via nonionic ethoxylated surfactants'
- DATABASE WPI Week 9133 Derwent Publications Ltd., London, GB; AN 91-242251 & JP,A,03 157 320 (SANPATSU SANGYO KK) , 5.Juli 1991
- Beilsteins Handbuch der Organischen Chemie, Band II, 2. Ergänzungswerk, S. 346f, Berlin 1942

## Beschreibung

Die Erfindung betrifft Öl-in-Wasser-Emulsionen mit speziellen Emulgatorkombinationen.

Eine Vielzahl von Mitteln werden heute als Öl-in-Wasser-Emulsionen formuliert. Sie bestehen dann in der Regel aus Wasser, Ölkomponente(n), Emulgator(en) sowie einer Reihe von weiteren, für den jeweiligen Verwendungszweck notwendigen Komponenten. Weiterhin ist es üblich, durch entsprechende Hilfsstoffe, z.B. Verdickungsmittel, die physikalischen Eigenschaften und das Erscheinungsbild der Emulsionen nach Wunsch einzustellen.

Aus einer Reihe von Gründen besteht heute vielfach das Ziel, die Rezepturen solcher Öl-in-Wasser-Emulsionen zu vereinfachen, das heißt, die gewünschten Produkte mit einer geringeren Zahl von Bestandteilen zu formulieren. Dies hat einerseits ökonomische Vorteile und kann andererseits in bestimmten Anwendungsbereichen, z. B. der Kosmetik, das Risiko mindern, daß empfindliche Personen und Allergiker Probleme mit einzelnen Komponenten des Mittels haben.

Es wurde nun gefunden, daß bei Verwendung spezieller Emulgatorkombinationen Öl-in-Wasser-Emulsionen mit hervorragenden Eigenschaften erhalten werden. Überraschenderweise kann bei diesen Emulsionen in vielen Fällen auf bestimmte Komponenten, z.B. spezielle Verdickungsmittel trotz hohen Wassergehalts, verzichtet werden. Weiterhin ist es in vielen Fällen ausreichend, die Emulgatoren lediglich in geringen Mengen, d.h. unter 1 Gew.-%. bezogen auf die Emulsion, einzusetzen.

Gegenstand der Erfindung ist daher eine Öl-in-Wasser-Emulsion, enthaltend 50 - 99 Gew.-% Wasser und 1 - 30 Gew.-% Ölphase, die dadurch gekennzeichnet ist, daß sie ein Emulgatorsystem (E) enthält, das besteht aus
(A) einem nichtionogenen Emulgator der Formel (I),

   ZₓR¹(R²-CO)_{y}G_{z} (I),

   in der Z steht für einen Zuckerrest, ausgewählt aus den Pentosen und Hexosen, x für eine Zahl von 1 bis 5, R¹ für einen gesättigten Alkylrest mit 1 bis 3 Kohlenstoffatomen, R² für einen linearen oder verzweigten Alkyloder ein- oder mehrfach ungesättigten Alkenylrest mit 8 bis 22 Kohlenstoffatomen, y für 1 oder 2, G für einen Polyglycerinrest, bestehend aus 2 bis 10 Glycerineinheiten, und z für 1 oder 2, und
(B) einem ionischen Emulgator, ausgewählt aus der Gruppe der kationischen und anionischen Emulgatoren, mit der Maßgabe, daß die Menge an ionischem Emulgator mindestens 0.1 Gew.-% bezogen auf die gesamte Emulsion beträgt, wenn es sich um einen anionischen Emulgator handelt.

Die nichtionogenen Emulgatoren (A) bestehen aus Bausteinen, die aus natürlichen, nachwachsenden Rohstoffen gewonnen werden. Sie weisen daher eine sehr gute biologische Abbaubarkeit auf und haben vorteilhafte ökologische Eigenschaften. Sie kommen daher dem Bestreben entgegen, Produkte auf Basis nachwachsender, natürlicher Rohstoffe zu formulieren.

Kernbaustein der Emulgatoren (A) ist ein Zuckerrest, ausgewählt aus den Hexosen und Pentosen. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Obwohl die Hexosen und Pentosen selbst bevorzugte Bausteine sind, ist es gewünschtenfalls auch möglich, Emulgatoren (E) erfindungsgemäß einzusetzen, die als Kernbaustein entsprechende Oligosaccharide enthalten. In einem solchen Fall ist es dann bevorzugt, daß die Oligosaccharide aus gleichen Grundbausteinen zusammengesetzt sind. Dabei sind die Oligosaccharide maximal aus 5 Zuckereinheiten aufgebaut.

Die Zuckerbausteine sind mit dem Alkylrest eines kurzkettigen Alkohols verethert sowie mit einer oder zwei langkettigen gesättigten oder ungesättigten Fettsäuren verestert. Als kurzkettiger Alkylrest kommen insbesondere der Methyl- und der Ethylrest in Betracht. Die Methylether sind dabei ganz besonders bevorzugt. Als langkettige Reste R² kommen lineare oder verzweigte Alkyl- oder ein- oder mehrfach ungesättigte Alkenylreste mit 8 bis 22 Kohlenstoffatomen in Betracht. Dabei sind solche Reste bevorzugt, die aus nachwachsenden Rohstoffen, wie Fetten oder Ölen, gewonnen werden können. Reste mit Kettenlängen von 10 bis 18 Kohlenstoffatomen sind dabei besonders bevorzugt. Bevorzugt sind Gruppen R²-CO, die sich beispielsweise von Laurin-, Myristin-, Palmitin-, Stearin-, Öl-, Linol- oder Linolensäure ableiten. Palmitoyl-, Oleoyl- und insbesondere Stearoylgruppen sind besonders bevorzugte Gruppen R²-CO. Weiterhin ist bevorzugt, daß die Emulgatoren (A) zwei, insbesondere zwei gleichartige, Acylgruppen R²-CO- enthalten. Es ist auch möglich, daß zur Herstellung der Emulgatoren (A) Mischungen von Fettalkoholen verwendet werden, die bei der Reduktion gängiger Fette oder Öle, wie zum Beispiel Kokosfett, Talg etc., anfallen. In diesem Fall kann R² auch für eine entsprechende Mischungen von Fettalkylresten stehen. Schließlich sind die Zuckerbausteine noch mit einem oder zwei Polyglycerinresten, die jeweils aus 2 bis 10 Glycerineinheiten, insbesondere 2 bis 5 Glycerineinheiten, bestehen, verethert.

Der ionische Emulgator (B) kann ein kationischer oder ein anionischer Emulgator sein.

Anionische Emulgatoren sind gekennzeichnet durch eine wasserlöslich machende, anionische Gruppe wie z. B. eine Carboxylat-, Sulfat-, Sulfonatoder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Emulgatoren sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Emulgatoren sind Alkylsulfate, Alkylpolyglykolethersulfate und Ethercarbonsäuren mit 10 bis 18 C-Atomen in der Alkylgruppe und bis zu 12 Glykolethergruppen im Molekül.

Bei den als ionische Emulgatoren eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den ionischen Emulgatoren, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden.

Die Verwendung von Produkten mit eingeengter Homologenverteilung kann bevorzugt sein.

Beispiele für erfindungsgemäß verwendbare kationische Emulgatoren sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Emulgatoren stellen die quaternisierten oder mit Aminogruppen derivatisierten Proteinhydrolysate dar, die beispielsweise unter den Warenzeichen Lamequat^{R} und Mackpro^{R} vertrieben werden.

Alkylamidoamine, insbesondere Fettsäureamidoamine wie das unter der Bezeichnung Tego Amid^{R}S 18 erhältliche Stearylamidopropyldimethylamin, zeichnen sich speziell durch ihre gute biologische Abbaubarkeit aus.

Ebenfalls erfindungsgemäß verwendbar sind die sehr gut biologisch abbaubaren quaternären Esterverbindungen, sogenannte "Esterquats", wie die unter den Warenzeichen Stepantex^{R} und Dehyquart^{R} vertriebenen Dialkylammoniummethosulfate und Methyl-hydroxyalkyldialkoyloxyalkyl-ammoniummethosulfate, wie z.B. N-Methyl-N,N,-bis(acyloxyethyl)-N(2-hydroxyethyl)ammoniummethosulfat sowie das unter dem Warenzeichen Mackalene^{R} vertriebene Isostearamidopropyl-morpholinlactat.

Erfindungsgemäß geeignet sind schließlich auch die kationischen Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 7232 (Hersteller: Th. Goldschmidt; diquaternäre Polydimethylsiloxane, Quaternium-80).

Ein Beispiel für ein als kationischer Emulgator einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Besonders bevorzugte kationische Emulgatoren sind quaternäre Ammoniumsalze, Alkylamidoamine und quaternäre Esterverbindungen.

Die nichtionogenen Emulgatoren (A) sind in den erfindungsgemäßen Öl-in-Wasser-Emulsionen bevorzugt in Mengen von 0,01 - 2,5 Gew.-%, insbesondere in Mengen von 0,05 - 1,0 Gew.-%, jeweils bezogen auf die gesamte Emulsion, enthalten.

Wenn es sich bei den ionischen Emulgatoren (B) um anionische Emulgatoren handelt, so sind diese bevorzugt in Mengen von 0,1 - 10 Gew.-%, insbesondere 0,5 - 5 Gew.-%, ebenfalls bezogen auf die gesamte Emulsion, enthalten.

Wenn es sich bei den ionischen Emulgatoren (B) um kationische Emulgatoren handelt, so sind diese bevorzugt in Mengen von 0,1 - 2 Gew.-%, insbesondere 0,4 - 0,8 Gew.-%, ebenfalls bezogen auf die gesamte Emulsion, enthalten.

Ebenfalls eine zwingende Komponente ist die Ölphase, die in den erfindungsgemäßen Emulsionen in Mengen von 1 - 30, insbesondere von 1 - 15 Gew.-%, bezogen auf die gesamte Emulsion, enthalten ist.

Als Ölkörper können beispielsweise verwendet werden:
- Mono-, Di- und Triglyceride und deren Mischungen,
- Paraffinöle,
- Fettalkohole,
- Fettalkylalkanolamide
- Silikonöle,
- Ester von Fettsäuren mit niederen Alkoholen,
- Ester von Fettsäuren mit Fettalkoholen und
- Dialkylether mit jeweils 6 bis 20 Kohlenstoffatomen in der Alkylkette.

Die erfindungsgemäßen Emulsionen können als Basis für kosmetische Mittel dienen. Als besonders vorteilhaft hat sich diese Basis zur Formulierung von Haut- und Haarbehandlungsmitteln erwiesen. Solche Mittel zeichnen sich dann durch ein gehaltvolles Aussehen und ein sehr gutes Fließverhalten aus. Ihre Viskosität kann bereits mit vergleichsweise geringen Emulgatormengen auf die gewünschten Werte eingestellt werden.

Diese Mittel können dann alle dem Fachmann bekannten Wirk-, Hilfs- und Zusatzstoffe enthalten. Diese sind in den dem Fachmann bekannten Monographien (z.B. K. Schrader, Grundlagen und Rezepturen der Kosmetik, Hüthig Buchverlag, Heidelberg) ausführlich dargestellt. Für Haarbehandlungsmittel sind dies beispielsweise
- zwitterionische Tenside, wie beispielsweise Betaine,
- ampholytische Tenside,
- nichtionogene Tenside, wie beispielsweise Alkylpolyglycoside und ethoxylierte Fettalkohole,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Methoimidazoliniumchlorid-Copolymere und quaternierter Polyvinylalkohol
- anionische Polymere wie beispielsweise Vinylacetat/Crotonsäure-Copolymere, Vinylacetat/Butylmaleat/Isobornylacrylat-Copolymere, Methylvinylether/Maleinsäureanhydrid-Copolymere und deren Ester sowie unvernetzte und mit Polyolen vernetzte Polyacrylsäuren,
- zwitterionische und amphotere Polymere wie beispielsweise Acrylamidopropyl-trimethylammoniumchlorid/Acrylat-Copolymere, Octylacrylamid/Methyl-methacrylat/tert.Butylaminoethylmethacrylat/2-Hydroxypropylmethacrylat-Copolymere,
- nichtionische Polymere wie beispielsweise Polyvinylpyrrolidon, Vinylpyrrolidon/Vinylacetat-Copolymere und Celluloseether,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Walrat, Bienenwachs und Montanwachs,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Bei erfindungsgemäßen Haarbehandlungsmitteln handelt es sich bevorzugt um Spülungen, Shampoos und Haarkuren. Die erfindungsgemäße Wirkstoffkombination kann aber auch in anderen Haarbehandlungsmitteln, wie z.B. Färbe- und Tönungsshampoos oder -cremes, Haarfärbemitteln, sowie in Rahmen einer Dauerwellbehandlung eingesetzt werden.

Die erfindungsgemäßen Haarbehandlungsmittel können sowohl auf dem Haar verbleiben. als auch nach einer gewissen Einwirkzeit, die in der Regel zwischen einigen Sekunden und ca. 20 Minuten liegt, wieder vom Haar abgespült werden.

Gegenstand der Erfindung ist daher auch die Verwendung der erfindungsgemäßen Öl-in-Wasser-Emulsion als kosmetisches Mittel zur Behandlung von Haut und/oder Haaren.

Die folgenden Beispiele sollen den Erfindungsgegenstand näher erläutern.

### Beispiele

### 1. Untersuchungen zur Bildung und Eigenschaften von Emulsionen

Es wurden folgende Komponenten eingesetzt:
Fett- bzw. Ölkörper:
   F1 : Paraffinöl perliquidum
   F2 : Cutina^{R} GMS¹
   F3 : Lanette^{R} 16²
Emulgatoren:
   A1 : Tego^{R} Care 450³
   AV : Aminol^{R} N⁴ (nicht erfindungsgemäß)

   B1 : Dehyquart^{R}A⁵
   B2 : Tego^{R}Amid S 18⁶
   B3 : Mackalene^{R} 426⁷
   B4 : Texapon^{R} N 25⁸

¹ Glycerinmonostearat (CTFA-Bezeichnung: Glyceryl Stearate) (HENKEL)
² C16-Fettalkohol (CTFA-Bezeichnung: Cetyl Alcohol) (HENKEL)
³ Methylglucosid-distearinsäureester, mit Polyglycerin verethert (CTFA-Bezeichnung(angemeldet): Polyglycerol Methyl Glucose Distearate) (GOLDSCHMIDT)
⁴ ethoxyliertes Fettsäuremonoethanolamid auf Basis Rapssamen (CTFA-Bezeichnung: PEG-4 Rapeseedamide) (CHEM-Y)
⁵ Trimethylhexadecylammoniumchlorid (CTFA-Bezeichnung: Cetrimonium Chloride; ca. 25 % Aktivsubstanz in Wasser) (HENKEL)
⁶ N,N-Dimethyl-N'-stearoyl-1,3-diamino-propan (CTFA-Bezeichnung: Stearamidopropyl Dimethylamin) (GOLDSCHMIDT)
⁷ Milchsäuresalz des Isostearamidopropylmorpholins (CTFA-Bezeichnung: Isostearamidopropylmorpholine Lactate; ca. 25 % Aktivsubstanz) (McINTYRE)
⁸ Natriumlaurylethersulfat (ca. 28 % Aktivsubstanz; CTFA-Bezeichnung: Sodium Laureth Sulfate) (HENKEL)

Tabelle 1 zeigt die erhaltenen Ergebnisse:

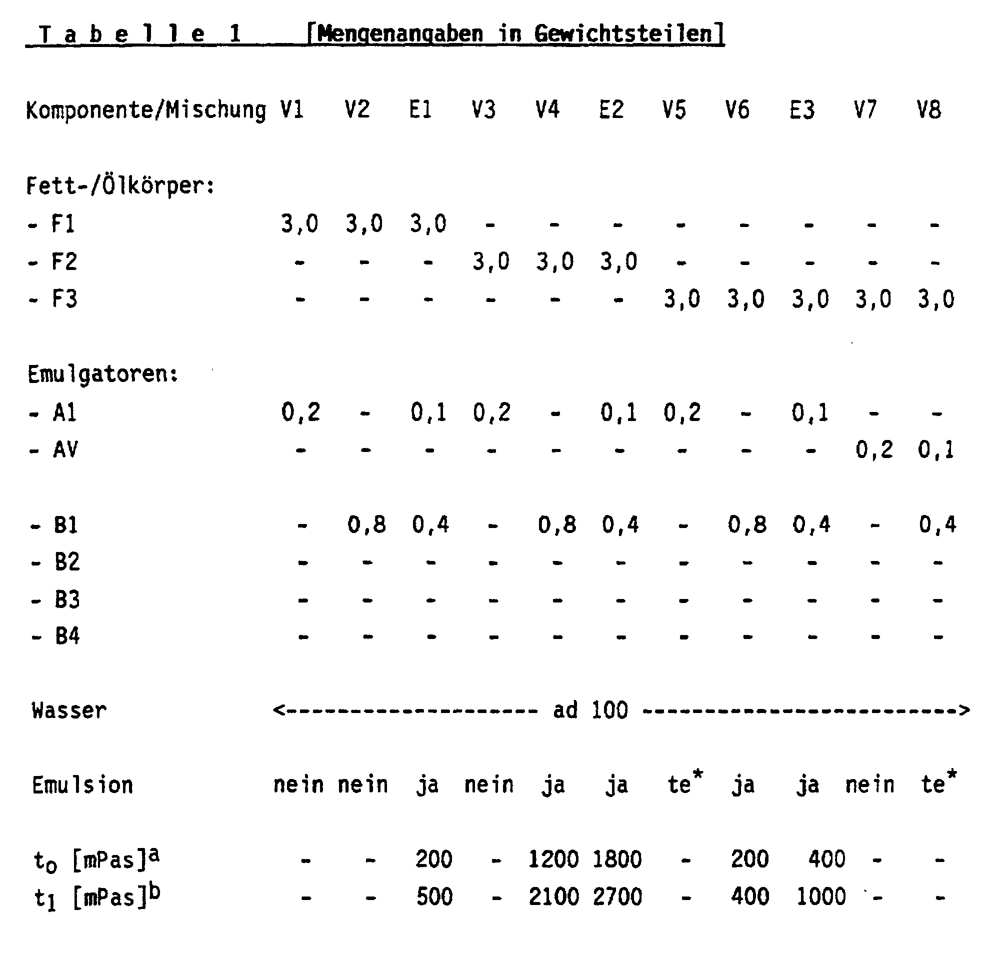

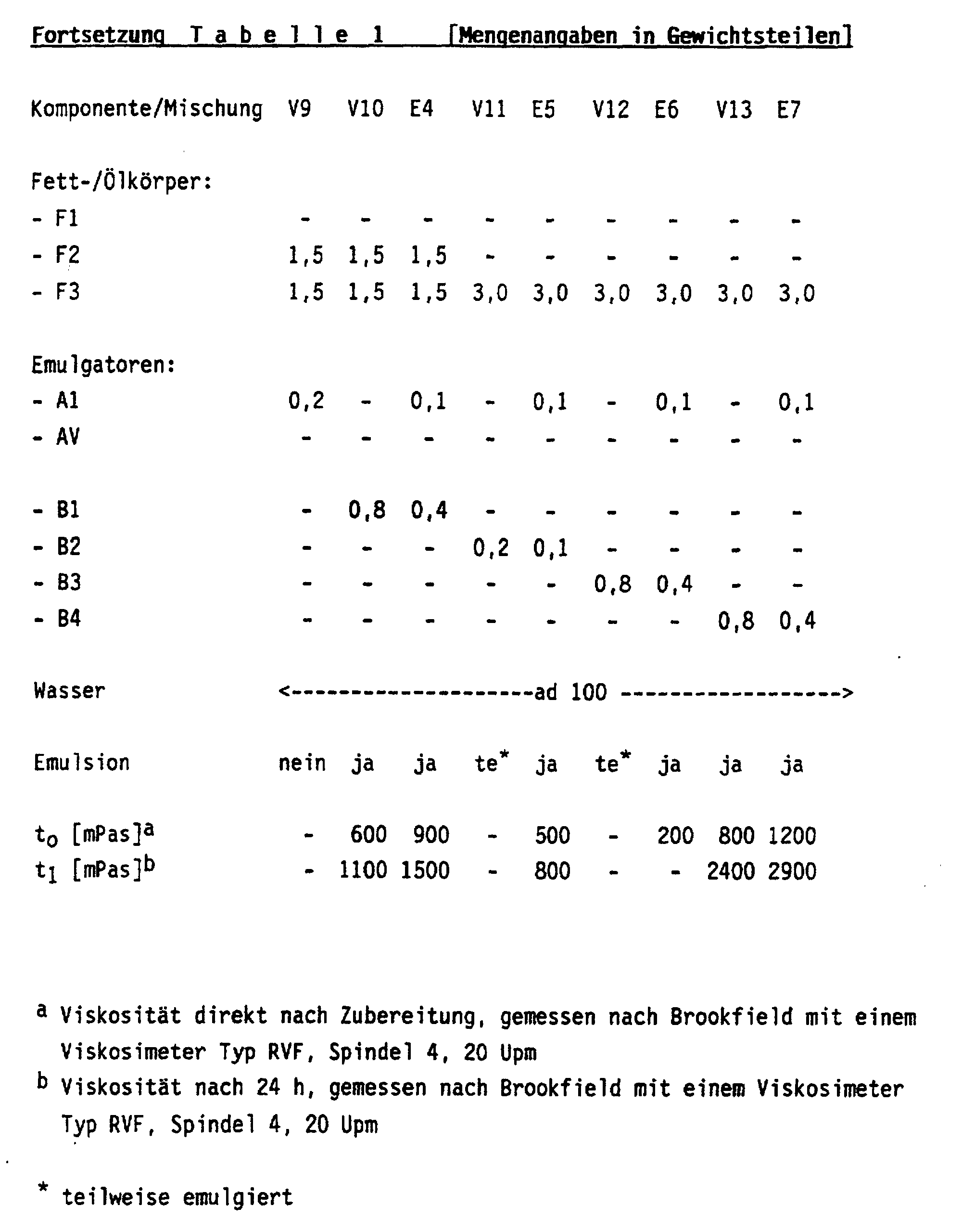

### 2. Anwendungsbeispiele

Alle Mengenangaben sind in Gewichtsteilen.

### 2.1. Haarkur

| | |
|---|---|
| Cutina^{R}GMS | 0,3 |
| Lanette^{R}16 | 4,8 |
| Paraffinöl perliq. | 3,9 |
| Cetiol^{R}OE⁹ | 0,2 |
| Tego Care^{R}450 | 0,4 |
| Dehyquart^{R}A | 3,0 |
| Dehyquart^{R}AU 46¹⁰ | 0,3 |
| Luviskol^{R}K 30¹¹ | 1,0 |
| Lamequat^{R}L¹² | 0,3 |
| Culminal^{R}¹³ MHPC 3000 | 0,6 |
| Konservierungsmittel | q.s. |
| Wasser | ad 100 |

| | |
|---|---|
| ⁹ Dioctylether (CTFA-Bezeichnung: Dicapryl Ether) (HENKEL) | |
| ¹⁰ N-Methyl-N,N-bis(acyloxyethyl)-N(2-hydroxyethyl)ammoniummethosulfat (ca. 90 % Aktivsubstanz in Isopropanol) (HENKEL) | |
| ¹¹ Polyvinylpyrrolidon (CTFA-Bezeichnung: PVP) (BASF) | |
| ¹² kationisiertes Kollagenhydrolysat (ca. 35 % Aktivsubstanz; CTFA-Bezeichnung: Lauryldimonium Hydroxypropyl Hydrolyzed Collagen) (HENKEL) | |
| ¹³ Methylhydroxypropylcellulose (AQUALON) | |

### 2.2. Reinigungsmilch

| | |
|---|---|
| Lanette^{R}16 | 3,0 |
| Cetiol^{R}SN¹⁴ | 2,5 |
| Texapon RN 2515 | 15,0 |
| Plantaren^{R}1200 UP¹⁶ | 3,0 |
| Tego Care^{R}450 | 0,15 |
| Myritol^{R}318¹⁷ | 1,8 |
| Proteol^{R}VS 22¹⁸ | 2,0 |
| Konservierungsmittel | q.s. |
| Wasser | ad 100 |

| | |
|---|---|
| ¹⁴ Cetylstearylisononanoat (CTFA-Bezeichnung: Cetearyl Isononanoate) (HENKEL) | |
| ¹⁵ Natriumlaurylethersulfat (ca. 28 % Aktivsubstanz; CTFA-Bezeichnung: Sodium Laureth Sulfate) (HENKEL) | |
| ¹⁶ C₁₂-C₁₆-Alkylglucosid mit Oligomerisationsgrad 1,4 (ca. 50 % Aktivsubstanz; CTFA-Bezeichnung: Lauryl Polyglycose) (HENKEL) | |
| ¹⁷ Fettsäuretriglycerid (CTFA-Bezeichnung: Caprylic Capric Triglyceride) (HENKEL) | |
| ¹⁸ Sojaproteinhydrolysat-Kokosfettsäure-Natriumsalz (ca. 22 % Aktivsubstanz; CTFA-Bezeichnung: Sodium Cocoyl Hydrolyzed Soy Protein) (SEPPIC) | |

### 2.3. Haarspülung

| | |
|---|---|
| Lanette^{R}16 | 3,0 |
| TegoAmid^{R}S 18¹⁹ | 1,2 |
| Dehyquart^{R}AU 46 | 0,5 |
| Tego Care^{R}450 | 0,2 |
| Natrosol^{R}250 HR²⁰ | 0,7 |
| Zitronensäure | 0,2 |
| Konservierungsmittel | q.s. |
| Wasser | ad 100 |

| | |
|---|---|
| ¹⁹ N,N-Dimethyl-N'-stearoyl-1,3-diamino-propan (CTFA-Bezeichnung: Stearamidopropyl Dimethylamin) (GOLDSCHMIDT) | |
| ²⁰ Hydroxyethylcellulose (AQUALON) | |

## Patentansprüche

1. Öl-in-Wasser-Emulsion, enthaltend 50 - 99 Gew.-% Wasser und 1 - 30 Gew.-% Ölphase, **dadurch gekennzeichnet, daß** sie ein Emulgatorsystem (E) enthält, das besteht aus
(A) einem nichtionogenen Emulgator der Formel (I),
ZₓR¹(R²-CO)_{y}G_{z} (I),
in der Z steht für einen Zuckerrest, ausgewählt aus den Pentosen und Hexosen, x für eine Zahl von 1 bis 5, R¹ für einen gesättigten Alkylrest mit 1 bis 3 Kohlenstoffatomen, R² für einen linearen oder verzweigten Alkyl- oder ein- oder mehrfach ungesättigten Alkenylrest mit 8 bis 22 Kohlenstoffatomen, y für 1 oder 2, G für einen Polyglycerinrest, bestehend aus 2 bis 10 Glycerineinheiten, und z = 1 oder 2, und
(B) einem ionischen Emulgator, ausgewählt aus der Gruppe der kationischen und anionischen Emulgatoren, mit der Maßgabe, daß die Menge an ionischem Emulgator mindestens 0.1 Gew.-% bezogen auf die gesamte Emulsion beträgt, wenn es sich um einen anionischen Emulgator handelt.

2. Öl-in-Wasser-Emulsion nach Anspruch 1, **dadurch gekennzeichnet, daß** der ionische Emulgator (B) ein kationischer Emulgator ist, der ausgewählt ist aus der Gruppe der quartären Ammoniumverbindungen, Alkylamidoamine und quaternären Esterverbindungen.

3. Öl-in-Wasser-Emulsion nach Anspruch 1, **dadurch gekennzeichnet, daß** der ionische Emulgator (B) ein anionischer Emulgator ist, der ausgewählt ist aus der Gruppe der Fettalkylpolyglykolethersulfate, Fettalkylsulfate und Fettalkylpolyglykolethercarboxylate.

4. Öl-in-Wasser-Emulsion nach einem der Ansprüche 1 bis 3, **dadurch gekennzeichnet, daß** in Formel (I) Z steht für Glucose, x = 1 ist, R¹ steht für eine Methylgruppe, R² steht für einen linearen oder verzweigten Alkyl- oder ein- oder mehrfach ungesättigten Alkenylrest mit 10 bis 18 Kohlenstoffatomen, y = 2 ist und G_{z} für einen Polyglycerinrest, bestehend aus 2 bis 5 Glycerineinheiten.

5. Öl-in-Wasser-Emulsion nach einem der Ansprüche 1 bis 4, **dadurch gekennzeichnet, daß** der Emulgator (A) in Mengen von 0,01 - 2,5 Gew.-%, bezogen auf die gesamte Emulsion, enthalten ist.

6. Öl-in-Wasser-Emulsion nach einem der Ansprüche 1 bis 5, **dadurch gekennzeichnet, daß** es sich um ein kosmetisches Mittel handelt.

7. Öl-in-Wasser-Emulsion nach einem der Ansprüche 1 bis 6, **dadurch gekennzeichnet, daß** das kosmetische Mittel ein Mittel zur Behandlung von Haut und/oder Haaren ist.

8. Verwendung einer Öl-in-Wasser-Emulsion nach Anspruch 7 zur Behandlung von Haut und/oder Haaren.

## Claims

1. An oil-in-water emulsion containing 50 to 99% by weight of water and 1 to 30% by weight of oil phase, **characterized in that** it contains an emulsifier system (E) which consists of
(A) a nonionic emulsifier corresponding to formula (I):
ZₓR¹(R²-CO)_{y}G_{z} (I)
in which Z is a sugar unit selected from pentoses and hexoses, x is a number of 1 to 5, R¹ is a saturated alkyl radical containing 1 to 3 carbon atoms, R² is a linear or branched alkyl radical or mono- or polyunsaturated alkenyl radical containing 8 to 22 carbon atoms, y is the number 1 or 2, G is a polyglycerol residue consisting of 2 to 10 glycerol units and z is the number 1 or 2, and
(B) an ionic emulsifier selected from the group of cationic and anionic emulsifiers, with the proviso that, in the case of an anionic emulsifier, the quantity of ionic emulsifier is at least 0.1% by weight, based on the emulsion as a whole.

2. An oil-in-water emulsion as claimed in claim 1, **characterized in that** the ionic emulsifier (B) is a cationic emulsifier selected from the group of quaternary ammonium compounds, alkylamidoamines and quaternary ester compounds.

3. An oil-in-water emulsion as claimed in claim 1, **characterized in that** the ionic emulsifier (B) is an anionic emulsifier selected from the group of fatty alkyl polyglycol ether sulfates, fatty alkyl sulfates and fatty alkyl polyglycol ether carboxylates.

4. An oil-in-water emulsion as claimed in any of claims 1 to 3, **characterized in that**, in formula (I), Z is glucose, x = 1, R¹ is a methyl group, R² is a linear or branched alkyl radical or mono- or polyunsaturated alkenyl radical containing 10 to 18 carbon atoms, y = 2 and G_{z} is a polyglycerol residue consisting of 2 to 5 glycerol units.

5. An oil-in-water emulsion as claimed in any of claims 1 to 4, **characterized in that** the emulsifier (A) is present in quantities of 0.01 to 2.5% by weight, based on the emulsion as a whole.

6. An oil-in-water emulsion as claimed in any of claims 1 to 5, **characterized in that** it is a cosmetic product.

7. An oil-in-water emulsion as claimed in any of claims 1 to 6 **characterized in that** the cosmetic product is a skin and/or hair treatment formulation.

8. The use of the oil-in-water emulsion claimed in claim 7 for the treatment of skin and/or hair.

## Revendications

1. Émulsion huile dans l'eau, renfermant 50 à 99 % en poids d'eau et 1 à 30 % en poids d'une phase huileuse, qui est **caractérisée en ce qu'**elle contient un système d'émulsifiants (E), qui est constitué de
(A) un émulsifiant non ionogène de la formule (I),
ZₓR¹(R²-CO)_{y}G_{z} (I),
dans laquelle Z représente un radical sucre sélectionné parmi les pentoses et les hexoses, x correspond à un nombre de 1 à 5, R¹ est un radical alkyle saturé comportant 1 à 3 atomes de carbone, R² équivaut à un radical alkyle linéaire ou ramifié ou à un radical alcényle mono ou polyinsaturé comportant 8 à 22 atomes de carbone, y correspond à 1 ou à 2, G représente un radical de polyglycérine constitué de 2 à 10 unités de glycérine, et Z est égal à 1 ou à 2, et de
(B) un émulsifiant ionique, sélectionné dans le groupe formé des émulsifiants cationiques et anioniques, à condition que la proportion d'émulsifiant ionique atteigne au moins 0,1 % en poids par rapport à la totalité de l'émulsion, s'il s'agit d'un émulsifiant anionique.

2. Émulsion huile dans l'eau selon la revendication 1, **caractérisée en ce que** l'émulsifiant (B) est un émulsif cationique, sélectionné dans le groupe des composés d'ammonium quaternaire, des alkylamidoamines et des composés d'esters quaternaires.

3. Émulsion huile dans l'eau selon la revendication 1, **caractérisée en ce que** l'émulsifiant (B) est un émulsif anionique, sélectionné dans le groupe des polyglycoléthersulfates d'alkyle gras, des sulfates d'alkyle gras et des polyglycoléthercarboxylates d'alkyle gras.

4. Émulsion huile dans l'eau selon une des revendications 1 à 3, **caractérisée en ce que** dans la formule (I), Z représente le glucose, x = 1, R¹ est un groupe méthyle, R² équivaut à un radical alkyle linéaire ou ramifié ou à un radical alcényle mono ou polyinsaturé comportant 10 à 18 atomes de carbone, y = 2 et G_{z} représente un radical de polyglycérine constitué de 2 à 5 unités de glycérine.

5. Émulsion huile dans l'eau selon une des revendications 1 à 4, **caractérisée en ce que** l'émulsifiant (A) est contenu en proportions de 0,01 à 2,5 % en poids par rapport à la totalité de l'émulsion.

6. Émulsion huile dans l'eau selon une des revendications 1 à 5, **caractérisée en ce qu'**il s'agit d'un produit cosmétique.

7. Émulsion huile dans l'eau selon une des revendications 1 à 6, **caractérisée en ce que** le produit cosmétique est un produit de traitement de la peau et/ou des cheveux.

8. Utilisation d'une émulsion huile dans l'eau selon la revendication 7 pour le traitement de la peau et/ou des cheveux.
